(19) Europäisches Patentamt

European Patent Office

Office européen des brevets

(11) Publication number: **0 538 500 B1**

# EUROPEAN PATENT SPECIFICATION

(45) Date of publication of patent specification: **01.02.95** (51) Int. Cl.⁶: **C07C 263/20**

(21) Application number: **91117877.0**

(22) Date of filing: **19.10.91**

(54) Process for the preparation of polymeric methylendiphenylene diisocyanate having reduced color and chloride content.

<table>
<tr><td>

(43) Date of publication of application:
**28.04.93 Bulletin 93/17**

(45) Publication of the grant of the patent:
**01.02.95 Bulletin 95/05**

(84) Designated Contracting States:
**AT BE CH DE DK ES FR GB IT LI NL SE**

(56) References cited:
**FR-A- 2 184 592**
**GB-A- 1 094 980**
**US-A- 4 465 639**
**US-A- 4 507 464**

</td><td>

(73) Proprietor: **BASF CORPORATION**
**8 Campus Drive**
**Parsippany,**
**New Jersey 07054 (US)**

(72) Inventor: **Reichel, Curtis John**
**12238 Fordline**
**Southgate,**
**Michigan 48195 (US)**
Inventor: **Speier, Jon Stanley**
**2658 Edgemont**
**Trenton, MI 48183 (US)**
Inventor: **Ott, Roger Alan**
**4826 Beech Harbor Avenue**
**Baton Rouge, LA 70817 (US)**

(74) Representative: **Michaelis, Wolfgang, Dr.**
**BASF Aktiengesellschaft,**
**Patentabteilung ZDX - C 6**
**D-67056 Ludwigshafen (DE)**

</td></tr>
</table>

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

**Description**

The subject invention pertains to the field of polymeric methylene diphenylene diisocyanate production by the phosgenation of polymeric methylene diphenylene diamines. More particularly, the subject invention pertains to a process for the reduction of color and chloride content in polymeric methylene diphenylene diisocyanates.

Methylene diphenylene diisocyanate (MDI) is produced by the phosgenation of methylene diphenylene diamine (MDA). MDA itself, and other like methylene diarylene diamines, is produced through the condensation of aniline or other aryl amines with formaldehyde. During the preparation of MDA and its analogues, a variety of isomers are formed, predominately the 4,4' isomer, with lesser quantities of the 2,4' isomer and 2,2' isomer. In addition to these two ring diamines, progressively lesser quantities of 3, 4, 5 and higher ring condensation products are also formed. These "polymeric MDAs" contain 3, 4, 5 or more amino groups respectively.

When this complex mixture of two ring and polymeric MDAs is phosgenated to form isocyanates, the corresponding isocyanates are produced. Thus the crude product contains, in progressively smaller quantities, two ring MDI consisting predominately of the 4,4'-, 2,4'-, and 2,2'-isomers; three ring triisocyanates, four ring tetraisocyanates, and so forth. The highest value product is 4,4'-MDI which has wide usefulness in preparing polyurethanes including polyurethane foams and elastomers. Mixtures containing both 2-ring and higher ring isocyanates are also useful. From a commercial perspective, it is desirable that all the isocyanates thus produced have low color and low chloride content. The presence of color in the MDI product is viewed by some as an indication of inferior product quality. Color also interferes with the production of colored polyurethanes and polyureas when dyes and pigments are added.

The chloride content of the MDI is also important, as the chlorine-containing impurities deactivate common amine-type catalysts used in the preparation of polyurethanes, and indeed affect the reactivity in general. Thus, the industry has long sought methods by which to minimize color and chloride content in polymethylene polyphenylene polyisocyanates.

The processing conditions and aryl amine/formaldehyde ratio can affect the color generated in any particular stage of the overall MDI process. However, in the final stages of the process, following phosgene stripping, the solvent, generally monochlorobenzene, is removed, as is approximately 5-10% of the isocyanate in the form of a two ring blend containing a major portion of 4,4'-MDI. Under the conditions of solvent removal and 2-ring isocyanate distillation, the crude isocyanate mixture is subjected to high temperatures for a extended period. Under these conditions, the color of the product darkens considerably. Once it has darkened, the color is most difficult to remove. Successive distillations under mild conditions may improve the color, but are time consuming and expensive.

In Great Britain patent 1,038,266, light colored polymeric MDI having low levels of chlorine-containing impurities is prepared by careful control of reactant ratios and mild production and purification techniques. These process requirements add substantial cost to the manufacturing process.

U.S. patent 4,465,639 produces light colored MDI through the addition of water to the MDI process stream in the presence of phosgene. However, in addition to reducing color and chloride content, the addition of water leads to higher carbodiimide content and additionally is corrosive to process equipment due to the presence of aqueous HCl.

In U.S. patent 4,507,464, polyether polyols or alkane polyols are added to the crude MDI prior to stripping of phosgene. However, this process results in the formation of urethane linkages and can result in increases in product viscosity. Furthermore, the preferred alkane polyol, glycerine, is but sparingly soluble in the isocyanate mixture, leading to processing problems.

Thus there continues to be a need for a cost-effective method of lowering color and chloride content without the drawbacks mentioned above.

These and other objects have been unexpectedly obtained through the addition of small quantities of a carboxylic acid to the phosgene stripper or the solvent removal column. The resulting product is much improved in color and in addition has improved chloride content.

Essentially any carboxylic acid may be used in the subject process, but preferably, low molecular weight carboxylic acids containing from 1 to 8 carbon atoms, most preferably formic acid, are utilized. Examples of other carboxylic acids which are suitable include acetic acid, propionic acid, 2-ethylhexanoic acid, benzylcarboxylic acid, oxalic acid. The acids are utilized in low quantities, i.e. less than 5% by weight relative to the isocyanate solution to which they are added, preferably 1% or less, and more preferably less than 0.2 or 0.1 weight percent.

In polymethylene polyphenylene polyisocyanate (PMDI) production, the polymethylene polyphenylene polyamines are generally phosgenated in a series of phosgenation reactors or columns, often operating at different temperatures. The phosgenation generally takes place in an inert solvent such as monochlorobenzene, dichlorobenzene, or diethylphthalate. Following phosgenation, excess phosgene is removed in a phosgene stripper which may be one or more of numerous types of distillation or evaporator vessels. The crude polyisocyanate solution exiting from the phosgene stripper has little free phosgene left, but does contain thermally decomposable chlorine-containing compounds such as carbamyl chlorides. Solvent removal takes place downstream from the phosgene stripper, following which distillation columns are used to purify the end product and separate it into the various commercial isocyanate blends. During solvent removal and distillation, sometimes with the aid of additional thermal treatment, isocyanate dimers (uretinediones) and a great part of the chlorine-containing impurities are thermally decomposed. Traces of these compounds remaining in the end product are responsible for the so-called easily hydrolyzable chlorides (EHC).

In the claims, the term "downstream from the phosgenation reactor" means a point in the process where the addition of phosgene has substantially ended, for example just prior to feeding the phosgenated product to the phosgene stripper.

The carboxylic acid is added to the isocyanate solution (isocyanate dissolved in process solvent) in or just prior to the phosgene stripper, or to the solvent removal column. Under these conditions, the phosgene content is 0.6 weight percent or less, preferably about 0.2 weight percent or less. The addition of the carboxylic acid to isocyanate blends which have already darkened is generally ineffective. Addition prior to the phosgene stripper may require the use of much greater amounts of carboxylic acid and thus may not be economically attractive. Most preferably, the carboxylic acid is added to the phosgene stripper, particularly to the bottoms to minimize phosgene contact.

For liquid carboxylic acids, these may be added neat to the process, or may be dissolved in process solvent. Solid carboxylic acids must generally be dissolved in order to facilitate handling.

The subject invention will now be illustrated by actual working examples. These examples should not be construed as limiting the scope of the invention in any way.

Examples 1-7, Control Examples A-D and Comparative Examples 1-2

Crude MDI dissolved in monochlorobenzene as taken from the bottoms of the phosgene stripper of a commercial MDI production process was treated with a variety of reagents in order to remove color. In procedure A, crude isocyanate containing approximately 13% MDI with the remainder being essentially monochlorobenzene solvent was added to a four neck flask equipped with an addition funnel and a Friedrichs condenser, and refluxed for 15 minutes after which the treating agent dissolved in monochlorobenzene was added and refluxed for an additional 15 minutes. The Friedrichs condenser was then removed and replaced with a short path distillation head following which the solvent was removed to the extent of 60% solvent concentration. Pot temperature during treating agent addition was that of the refluxing solvent, approximately 135-145°C, and addition took place over 30 minutes. Under these condition, the phosgene content was calculated to be approximately 0.04 weight percent. Residual solvent was then removed in a rotary evaporator at the indicated temperature, followed by thermal treatment, mimicking the industrial process, at the indicated temperature and = 13,2 mbar (10mm Hg) pressure for 15 minutes. The results are summarized in Table 1.

In procedure B, to stripper process bottoms containing c.a. 13% PMDI was added the treating agent in the quantity specified in Table 1. The solution was then evaporated in a rotary evaporator maintained at the indicated temperature until only traces (1-2 weight percent) of monochlorobenzene.

Gardner color was assessed against pure monochlorobenzene in a Hellige Comparator in accordance to ASTM method D1544. Iodine values were measured by a method similar to DIN 6162. Easily hydrolyzable chloride (EHC) was measured by standard tests. The actual test methods used are unimportant, as the relative values obtained by whichever test methods are utilized serve to characterize the results.

3

**TABLE 1**

| Example | Solvent Evaporation Temperature, °C | Procedure | Treating Agent @ wt% | $I_2$ Color | Gardner Color | EHC, ppm |
|---|---|---|---|---|---|---|
| Control A | 120 | B | none | 23 | 7.5 | 235 |
| Control B | 165 | B | none | 23 | 7.5 | 205 |
| Control C | 150 | A | none | 50 | --- | 420 |
| Control D | 165 | B | none | 30 | 8.5 | 276 |
| Example 1A | 120 | B | $H_2CO_2$ @ 0.2 | 18 | 6.5 | 199 |
| Example 2B | 165 | B | $H_2CO_2$ @ 0.2 | 18 | 6.5 | 180 |
| Example 3B | 165 | B | $H_2CO_2$ @ 0.2 | 25 | 7.5 | 62 |
| Example 4B | 165 | B | $H_2CO_2$ @ 1.0 | 15 | 6.5 | 124 |
| Example 5D | 165 | B | EHA[1] @ 0.2 | 20 | 7.0 | 291 |
| Example 6D | 165 | B | $H_2CO_2$ @ 0.2 | 23 | 7.5 | 174 |
| Example 7C | 150 | A | $H_2CO_2$ @ 0.12 | 30 | --- | 186 |
| Comparative 1C | 150 | A | pg[2] @ 0.12 | 36 | --- | 412 |
| Comparative 2C | 150 | A | DEG[3] @ 0.12 | 40 | --- | 268 |

[1] 2-ethylhexanoic acid
[2] polypropylene glycol
[3] diethylene glycol

In Table 1, examples within the scope of the subject invention are labeled in accordance with the sample isocyanates used in controls A-D. Thus example 1A utilized the same isocyanate as control A while example 5D utilized the same isocyanate as Control D. The same nomenclature is utilized with the Comparative Examples.

4

EP 0 538 500 B1

**Claims**

1. A process for the production of polymethylene polyphenylene polyisocyanates through the phosgenation of polymethylene polyphenylene polyamines in one or more phosgenation reactors following which excess phosgene is removed from the crude reaction mixture in a phosgene stripper, characterised by :

   adding to the process at a point downstream from the last phosgenation reactor, a carboxylic acid in an amount less than 5 weight percent relative to the process liquor to which the acid is added, and which is effective to reduce the color and/or hydrolyzable chloride content of the polyisocyanate.

2. The process of claim 1 wherein said carboxylic acid is a carboxylic acid containing from 1 to 8 carbon atoms.

3. The process of claim 1 wherein said carboxylic acid is formic acid.

4. The process of claim 1 to 3 wherein the carboxylic acid is added in an amount of 1 weight percent or less.

5. The process of claim 1 to 3 wherein the carboxylic acid is added in an amount of 0.2 weight percent or less.

6. The process of claim 1 wherein the carboxylic acid is added to the process downstream from the phosgene stripper but prior to the point at which significant color would be developed if the carboxylic acid were not added to the process.

7. The process of claim 1 wherein the carboxylic acid is added to a solvent removal column in the polyisocyanate production process.


**Patentansprüche**

1. Verfahren zur Herstellung von Polymethylenpolyphenylen-polyisocyanaten durch Phosgenierung von Polymethylen-polyphenylen-polyaminen in einem oder mehreren Phosgenierungsreaktoren, wobei überschüssiges Phosgen aus der Rohreaktionsmischung mittels eines Phosgenstrippers entfernt wird, dadurch gekennzeichnet, daß man :

   eine Carbonsäure an einer dem letzten Phosgenierungsreaktor nachgeschalteten Stelle in einer Menge von 5 Gewichtsprozent, bezogen auf die Verfahrensflüssigkeit, in die die Säure eingebracht wird, einbringt, mit der die Farbe und/oder der hydrolysierbare Chloridgehalt des Polyisocyanats wirksam vermindert wird.

2. Verfahren nach Anspruch 1, dadurch gekennzeichnet, daß die Carbonsäure 1 bis 8 Kohlenstoffatome aufweist.

3. Verfahren nach Anspruch 1, dadurch gekennzeichnet, daß es sich bei der Carbonsäure um Ameisensäure handelt.

4. Verfahren nach Anspruch 1 bis 3, dadurch gekennzeichnet, daß die Carbonsäure in einer Menge von 1 Gewichtsprozent oder weniger zugegeben wird.

5. Verfahren nach Anspruch 1 bis 3, dadurch gekennzeichnet, daß die Carbonsäure in einer Menge von 0,2 Gewichtsprozent oder weniger zugegeben wird.

6. Verfahren nach Anspruch 1, dadurch gekennzeichnet, daß die Carbonsäure in das Verfahren an einer dem Phosgenstripper nachgeschalteten Stelle, aber vor der Stelle, an der ohne Einbringung der Carbonsäure in das Verfahren eine deutliche Färbung entstehen wurde, eingebracht wird.

7. Verfahren nach Anspruch 1, dadurch gekennzeichnet, daß die Zugabe der Carbonsäure in eine Kolonne zur Lösungsmittelabtrennung beim Polyisocyanat-Herstellungsverfahren erfolgt.

5

**Revendications**

1. Procédé pour la production polyisocyanates de polyméthylène-polyphénylène par la phosgénation de polyméthylène-polyphénylène-polyamines dans un ou plusieurs réacteurs de phosgénation, à la suite de laquelle l'excès de phosgène est éliminé du mélange réactionnel brut dans un extracteur de phosgène, caractérisé par :

   l'addition au procédé, en un point situé en aval du dernier réacteur de phosgénation, d un acide carboxylique en uns quantité inférieure à 5 pour cent en poids par rapport à la liqueur traitée à laquelle l'acide est ajouté, et qui est efficace pour réduire la couleur et/ou la teneur en chlorure hydrolysable du polyisocyanate.

2. Procédé de la revendication 1, dans lequel ledit acide carboxylique est un acide carboxylique contenant 1 à 8 atomes de carbone.

3. Procédé de la revendication 1, dans lequel ledit acide carboxylique est l'acide formique.

4. Procédé de la revendication 1 à 3, dans lequel l'acide carboxylique est ajouté en une quantité de 1 pour cent en poids ou moins.

5. Procédé de la revendication 1 à 3, dans lequel l'acide carboxylique est ajouté en une quantité de 0,2 pour cent en poids ou moins.

6. Procédé de la revendication 1, dans lequel l'acide carboxylique est ajouté au procédé en aval de l'extracteur de phosgène, mais avant le point où une couleur appréciable se développerait si l'acide carboxylique n'était pas ajouté au procédé.

7. Procédé de la revendication 1, dans lequel l'acide carboxylique est ajouté à une colonne d'élimination de solvant dans le procédé de production de polyisocyanate.